# EUROPEAN PATENT SPECIFICATION

(11) **EP 4 037 569 B1**
(45) Date of publication and mention of the grant of the patent: **19.03.2025**
(21) Application number: 20780679.5
(22) Date of filing: 25.09.2020
(51) Int. Cl.: A61B 8/08, A61B 8/00, A61B 8/14, G16H 50/20

(54) **RECORDING ULTRASOUND IMAGES**
AUFNAHME VON ULTRASCHALLBILDERN
ENREGISTREMENT D'IMAGES ULTRASONORES

(30) Priority: 30.09.2019 US 201962908118 P
(43) Date of publication of application: 10.08.2022
(73) Proprietor: Koninklijke Philips N.V., 5656 AG Eindhoven (NL)
(72) Inventor: ROUNDHILL, David Nigel, 5656 AE Eindhoven (NL); KLINDER, Tobias, 5656 AE Eindhoven (NL); SCHMIDT-RICHBERG, Alexander, 5656 AE Eindhoven (NL); LENGA, Matthias, 5656 AE Eindhoven (NL); ORASANU, Eliza Teodora, 5656 AE Eindhoven (NL); LORENZ, Cristian, 5656 AE Eindhoven (NL)
(74) Representative: Philips Intellectual Property & Standards
(86) International application number: PCT/EP2020/076833
(87) International publication number: WO 2021/063807

(56) References cited:
- EP-A1- 3 513 738
- WO-A1-2020/239842
- US-A1- 2011 172 536
- US-A1- 2014 128 739
- US-A1- 2014 187 946
- US-A1- 2015 305 718
- US-A1- 2015 351 725
- US-A1- 2017 128 045

## Description

### TECHNICAL FIELD

The disclosure herein relates to ultrasound imaging. Particularly, but non-exclusively, embodiments herein relate to systems and methods for recording ultrasound images.

### BACKGROUND

US 2015/351725 discloses an ultrasound system which two-dimensional images are processed to derive a three-dimensional image. A segment region is selected for rescanning using setting marks.

US 2014/187946 discloses an ultrasound system which identifies an object of interest in an image. The image acquisition parameters are adjusted based on changes in position and/or orientation of the object of interest.

WO 2020/239842 discloses an ultrasound imaging system for generating volumetric images. It can switch from a 2D imaging mode to a volumetric imaging mode based on identification of a region of interest.

Ultrasound imaging is used in a range of medical applications such as, for example, fetal monitoring. Medical ultrasound imaging involves moving a probe comprising an ultrasound transducer that produces high frequency sound waves over the skin. The high frequency sound waves traverse through the tissue and reflect off internal surfaces (e.g. tissue boundaries). The reflected waves are detected and used to build up an image of the internal structures of interest.

Ultrasound imaging can be used to create two- or three- dimensional images. In a typical workflow, a user (e.g. sonographer, radiologist, clinician or other medical professional) may use two-dimensional imaging to locate an anatomical feature of interest. Once the feature is located in two dimensions, the user may activate a three-dimensional mode to take a three-dimensional image.

Imaging in this way is highly specialized and often requires many years of training. Efficient and accurate three-dimensional image acquisition methods that integrate well into the workflow are therefore desirable. It is an object of the disclosures herein to develop systems and methods to aid users, for example, to enable less experienced clinicians (such as, for example, primary care physicians), to be able to record three-dimensional ultrasound images in an accurate manner.

### SUMMARY

The invention is defined by the independent claims. Dependent claims define advantageous embodiments.

As described above, ultrasound image capture is highly specialized. In a typical workflow, a feature (e.g. anatomical feature) of interest is located by a user using two dimensional ultrasound imaging. Once the feature of interest is located, a three-dimensional imaging mode may be activated, to take the three-dimensional image. In some known methods, the user, after having located the feature of interest in the two-dimensional image, then has to activate the three-dimensional mode, for example, by manually clicking a button or interacting with a user screen.

Activating a three-dimensional imaging mode in this way may be distracting. Furthermore, the user may inadvertently move the ultrasound transducer away from the feature of interest when activating the three-dimensional imaging mode. Manual triggering of the three-dimensional imaging mode can therefore lead to non-optimal acquisitions, in which, for example, the target anatomy is only partially visible.

It is an object of the embodiments herein to improve upon such existing methods of recording ultrasound images.

Thus, according to a first aspect, there is a system according to claim 1.

In this manner, the three-dimensional imaging mode is triggered automatically after the predetermined time interval without the user having to manually intervene. Furthermore, the alert provides the user with prior warning that the three-dimensional image capture is about to commence, such that the user may terminate the three-dimensional image capture during the predetermined time interval (e.g. before the three-dimensional image capture is commenced). Because the user does not have to manually intervene, the user can concentrate solely on the position of the ultrasound transducer throughout, leading to a smoother workflow and better-quality ultrasound images.

According to a second aspect there is a computer implemented method of recording ultrasound images according to claim 13.

According to a third aspect there is a computer program product according to claim 14.

### BRIEF DESCRIPTION OF THE DRAWINGS

For a better understanding and to show more clearly how embodiments herein may be carried into effect, reference will now be made, by way of example only, to the accompanying drawings, in which:
Fig. 1 is a schematic of a system according to some example embodiments herein;
Fig. 2 shows an example method according to some embodiments herein;
Fig. 3 shows an example display according to some embodiments herein; and
Fig. 4 shows an example ultrasound system according to some embodiments herein.

### DETAILED DESCRIPTION

As described above, when imaging a feature of interest such as an anatomical feature in three-dimensional ultrasound, a work-flow is often followed whereby the user (e.g. clinician or medial professional) determines an appropriate position for the ultrasound probe e.g. where the probe should be positioned relative to the patient's body, using two dimensional ultrasound. Once an appropriate position is determined, the user then often has to (manually) activate the three-dimensional ultrasound image acquisition mode in order to record the three-dimensional ultrasound image. It is an object of embodiments herein to improve upon such systems and methods.

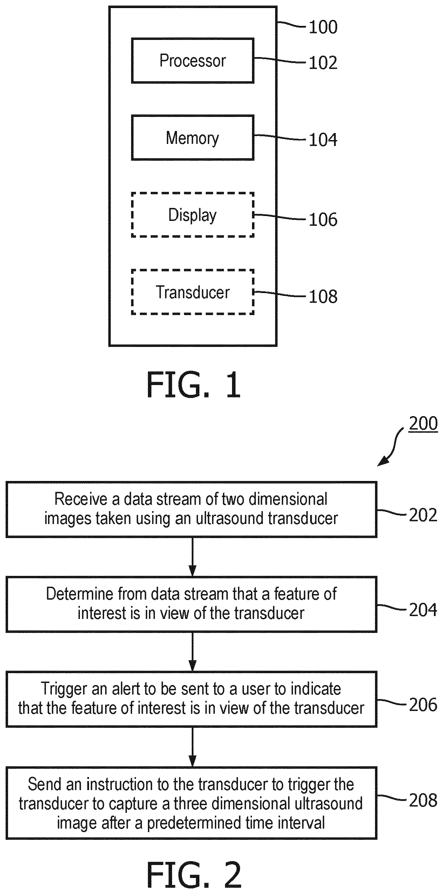

Fig. 1 illustrates a system (e.g. apparatus) 100 for recording ultrasound images according to some embodiments herein. The system 100 is for recording (e.g. acquiring or taking) ultrasound images. The system 100 may comprise or be part of a medical device such as an ultrasound system.

With reference to Figure 1, the system 100 comprises a processor 102 that controls the operation of the system 100 and that can implement the method described herein. The processor 102 can comprise one or more processors, processing units, multi-core processors or modules that are configured or programmed to control the system 100 in the manner described herein. In particular implementations, the processor 102 can comprise a plurality of software and/or hardware modules that are each configured to perform, or are for performing, individual or multiple steps of the method described herein.

Briefly, the processor 102 of the system 100 is configured to receive a data stream of two dimensional images taken using an ultrasound transducer, determine from the data stream that a feature of interest is in view of the transducer, trigger an alert to be sent to a user to indicate that the feature of interest is in view of the transducer, and send an instruction to the transducer to trigger the transducer to capture a three dimensional ultrasound image after a predetermined time interval.

Technically, this may provide an improved manner in which to trigger automated capture of three-dimensional ultrasound images by providing a predetermined time period in which the user may, for example, abort or refine the automatic image capture. Some embodiments may therefore enable human-in-the-loop automation (e.g. automation with human oversight) which may improve the interaction between human and automation process in order to produce improved three-dimensional ultrasound image capture of a feature of interest.

In some embodiments, as illustrated in Figure 1, the system 100 may also comprise a memory 104 configured to store program code that can be executed by the processor 102 to perform the method described herein. Alternatively, or in addition, one or more memories 104 may be external to (i.e. separate to or remote from) the system 100. For example, one or more memories 104 may be part of another device. A memory 106 can be used to store images, information, data, signals and measurements acquired or made by the processor 102 of the system 100 or from any interfaces, memories or devices that are external to the system 100.

In some embodiments, as illustrated in Fig. 1, the system 100 may further comprise a transducer 108 for capturing ultrasound images. Alternatively, or additionally, the system 100 may receive (e.g. via a wired or wireless connection) a data stream of two dimensional images taken using an ultrasound transducer that is external to the system 100.

The transducer may be formed from a plurality of transducer elements. Such transducer elements may be arranged to form an array of transducer elements. The transducer may be comprised in a probe such as a handheld probe that can be held by a user (e.g. sonographer, radiologist or other clinician) and moved over a patient's skin. The skilled person will be familiar with the principles of ultrasound imaging, but in brief, ultrasound transducers comprise piezoelectric crystals that can be used both to generate and detect/receive sound waves. Ultrasound waves produced by the ultrasound transducer pass into the patient's body and reflect off the underlying tissue structures. Reflected waves (e.g. echoes) are detected by the transducer and compiled (processed) by a computer to produce an ultrasound image of the underlying anatomical structures, otherwise known as a sonogram.

In some embodiments the transducer may comprise a matrix transducer that may interrogate a volume space.

In some embodiments, as illustrated in Fig. 1, the system 100 may also comprise at least one user interface such as a user display 106. The processor 102 may be configured to control the user display 106 to display or render, for example, portions of the received data stream or ultrasound images and/or the alert to the user. The user display 106 may comprise a touch screen or an application (for example, on a tablet or smartphone), a display screen, a graphical user interface (GUI) or other visual rendering component.

Alternatively, or in addition, at least one user display 106 may be external to (i.e. separate to or remote from) the system 100. For example, at least one user display 106 may be part of another device. In such embodiments, the processor 102 may be configured to send an instruction (e.g. via a wireless or wired connection) to the user display 106 that is external to the system 100 in order to trigger (e.g. cause or initiate) the external user displays to display the alert to the user to indicate that the feature of interest is in view of the user.

It will be appreciated that Figure 1 only shows the components required to illustrate this aspect of the disclosure, and in a practical implementation the system 100 may comprise additional components to those shown. For example, the system 100 may comprise a battery or other means for connecting the system 100 to a mains power supply. In some embodiments, as illustrated in Figure 1, the system 100 may also comprise a communications interface (or circuitry) 108 for enabling the system 100 to communicate with any interfaces, memories and devices that are internal or external to the system 100, for example over a wired or wireless network.

In more detail, the system 100 receives a data stream (e.g. continuous sequence) of two dimensional images taken using an ultrasound transducer 108. As noted above, the ultrasound transducer may be integral to the system 100, or alternatively, separate to the system 100.

The data stream comprises a live or real-time data stream of two-dimensional ultrasound images or ultrasound image data. In this sense, the data stream is produced and received by the system in real time, e.g. as the user records or takes the data with the ultrasound transducer.

In some embodiments, a data stream of ultrasound "rf" (radio frequency) data may be received and converted by the processor 102 into a stream (e.g. sequence) or 2D images. As will be familiar to the skilled person, a 2D image may be formed from rf image data by beamforming, signal processing and scan converting such rf data.

The processor 102 may be further configured to send an instruction to a display, such as user display 106 to instruct the user display 106 to display or render two-dimensional ultrasound images comprised in the data stream to the user.

The processor 102 is configured to determine from the data stream that a feature of interest is in view of the transducer. In some embodiments, the data stream comprises medical (or veterinary) image data and the feature of interest may comprise, for example a particular anatomical feature. The anatomical feature may comprise any anatomical feature, such as, for example, a ventricle or valve in the heart.

In some embodiments, the processor may determine that the feature of interest is in view of the transducer if the feature of interest if the feature of interest is in an appropriate orientation or suitably positioned in the field of view of the transducer in order to record a suitable three-dimensional ultrasound image.

In some embodiments, the processor is configured to determine, from the data stream, that a feature of interest is in view of the transducer using a machine learning model that has been trained to identify the feature of interest in two-dimensional ultrasound image data.

The machine learning model may take as input the data stream of two-dimensional ultrasound image data and output a classification or indication of the contents of each frame or image in the data stream as that frame or image is received by the system (e.g. the anatomical features visible in each two dimensional image). Alternatively, in some embodiments, the machine learning model may only provide an output when a particular feature (e.g. the feature of interest) is comprised in a frame of the data stream.

The skilled person will be familiar with machine learning models and methods for identifying (e.g. classifying) objects in images. An example may be found in the paper Joseph Redmon, Santosh Divvala, Ross Girshick, Ali Farhadi: You Only Look Once: Unified, Real-Time Object Detection, 2016. The "You Only Look Once" (YOLO) system uses neural networks to classify objects in images. As reported in the reference, trained models such as YOLO may be used to process data streams comprising sequences of images in real-time at rates of between 45 and 155 frames per second.

The skilled person will appreciate that trained neural networks are just one example of a type of model that may be used herein and that any type of machine learning model that can be trained to take as input a two-dimensional ultrasound image and provide as output a classification of the contents of the image, may be used in embodiments herein. Examples of such machine learning models include, but are not limited to, convolutional neural networks and random forest models.

In some embodiments the machine learning model may be trained to determine that the feature of interest is in view of the transducer in such as manner that a three-dimensional ultrasound image may be captured that is suitable for medical diagnostic purposes. For example, the machine learning model may determine that not only is the feature of interest in view of the transducer, but that it is in an appropriate orientation, or position within the field of view of the transducer in order for the medical diagnostic to be made. This may be achieved by training the machine learning model with training data comprising: i) example images of features of interest, and ii) ground truth labels indicating whether the image is suitable or not suitable for the medical diagnostic to be made.

In some clinical workflows, the user or radiologist is required to capture ultrasound images of a range or sequence of anatomical features. As an example, a medical protocol may dictate that during a neonatal scan, the user or radiologist must image a particular set of anatomical features of the fetus, or make a particular set of measurements. For example, a medical protocol may stipulate for example, that the brain of the fetus be imaged and each valve in the heart.

As such, in some embodiments, the processor may be further configured to receive an indication from the machine learning model that a potential feature is in view of the transducer, and determine whether the potential feature comprises a feature of interest by comparing the potential feature to a list of features of interest. As noted above, the list of features of interest may be derived from a medical protocol describing a plurality of anatomical features to be captured in the three-dimensional ultrasound image according to the protocol.

Alternatively, in some embodiments, the machine learning model may be trained to recognize those (e.g. only those) anatomical features comprised in a medical protocol. In some embodiments, a different machine learning model may thus be configured for each medical protocol.

Once it has been determined from the data stream that a feature of interest is in view of the transducer, the processor is caused to trigger an alert to be sent to a user to indicate that the feature of interest is in view of the transducer.

In some embodiments, the processor may send an instruction to the user display 106 to display the alert.

The alert may comprise a visual indication that the feature of interest is in view of the transducer. For example, in some embodiments, the alert may comprise text describing the feature of interest. For example, if the feature of interest comprises a fetal head, the alert may comprise the words "fetal head" appearing on the user display (e.g. to the side of, or over part of the two dimensional ultrasound image being viewed by the user.)

In some embodiments, the alert may comprise an indication of the time remaining before the three-dimensional ultrasound image capture will be commenced. For example, the alert may comprise a countdown of the time remaining before the auto-commencement of the three-dimensional ultrasound image capture.

In some embodiments, the alert may comprise, for example, a progress bar (or reverse progress bar) that counts down the time remaining. The progress bar may be in the form of an "egg timer".

In some embodiments, the alert may comprise an audible alert. For example, such as a beep, or audible instructions informing the user that the feature of interest is in view of the transducer. In this way, the user may be alerted to the fact that the feature of interest is in view and prompted, for example, to hold the transducer steady in order to facilitate the three-dimensional image capture.

As noted above, the processor 102 is also configured to send an instruction to the transducer to trigger the transducer to capture a three-dimensional ultrasound image after a predetermined time interval. Put another way, the processor is configured to initiate (e.g. automatically) a recording of a three dimensional image after a set time delay.

In some embodiments, the predetermined time interval may be configurable according to the user's preferences.

The predetermined time interval provides the user with a time window in which the user has advance warning that the three dimensional image capture is going to commence, e.g. so that the user may hold the probe still, or to make small adjustments before three dimensional image capture starts.

The predetermined time interval also enables the user to terminate the three-dimensional image capture before it starts, if desired. For example, if the user does not want a three-dimensional image to be recorded at that moment, then the user may manually indicate (e.g. by pressing or clicking a button) that the three dimensional image capture should be halted or aborted.

In other embodiments, the user may terminate the recording of the three-dimensional ultrasound image by moving the ultrasound probe. For example, in some embodiments, the system 100 may further comprise an ultrasound probe comprising transducer 106 and a motion sensor. The processor may be configured to receive motion data relating to motion of the transducer (e.g. data indicating whether, or the manner in which, the transducer is being moved). The processor may be further configured to send the instruction to the transducer (or not send the instruction to the transducer) based on the received motion data.

The data stream of two-dimensional ultrasound images is analyzed in order to detect motion. For example, motion data may be determined by analyzing the two-dimensional data stream and determining movement of the image content. Large variations of the image content may indicate motion (e.g. indicating that the user is still in the process of navigating to an optimal position). Lower variations in the image contents may indicate that the transducer is being held still.

In some embodiments, the processor may be configured to send the instruction to the transducer to initiate the three-dimensional imaging mode if the motion data indicates that the transducer has been moved by less than a threshold distance during the predetermined time interval. For example, if the user has held the probe or transducer still (e.g. to within the threshold distance) whilst the alert has been displayed to them, then this may be taken to be an indication that the user is ready/happy for three dimensional ultrasound imaging to be commenced.

Thus, the system simultaneously communicates to the user that they are in the proximate location of the desired view and, as long as they stay in such a proximate location, a one-time three-dimensional image acquisition will automatically proceed shortly after a given time interval without the need of further interaction (e.g. without explicit triggering).

In some embodiments, additionally or alternatively, the processor may be further configured to refrain from sending the instruction to the transducer if the motion data indicates that the transducer has been moved by more than the threshold distance during the predetermined time interval. For example, if the alert is displayed to the user that three-dimensional image capture is about to be commenced, the user may move or disengage the probe from the surface of the patient's skin in order to terminate the three-dimensional image capture.

In this way, the user may indicate, during the predetermined time interval between receiving the alert and the commencement of the three-dimensional image capture, that they do not want the three-dimensional imaging mode to be initiated by moving the transducer. This workflow (initiating three-dimensional ultrasound image capture unless indicated otherwise) is more user friendly and streamlined compared to methods whereby the user initiates three-dimensional image capture manually. It also allows the user to concentrate on finding and holding the transducer in the correct place without distractions or inadvertent movements as might be present if they were to manually initiate the three-dimensional mode.

In some embodiments, the alert may disappear when three when three-dimensional mode is not selected (e.g. if the probe is moved by the user during the predetermined time period).

In summary, embodiments herein allow a user to get a clear visible signal that three-dimensional imaging is to start, without diverting their gaze from the main display. Furthermore, the user knows when to maintain the transducer position. The user knows that three-dimensional imaging mode will start at a predictable moment in time according to the displayed timer/progress bar. In embodiments where the three-dimensional imaging model is triggered based on motion of the transducer 108, as the motion has to be frozen (e.g. probe held still) to trigger 3D acquisition, the likelihood of unintended motion during the three dimensional image capture is also reduced.

Turning now to Fig. 2, in some embodiments there is a computer implemented method 200 of recording ultrasound images. The method 200 may be performed, for example, by the system 100 described above. In a first block 202, the method 200 comprises receiving a data stream of two-dimensional images taken using an ultrasound transducer 108. In a second block 204, the method 200 comprises determining from the data stream that a feature of interest is in view of the transducer 108. In a third block 206 the method comprises triggering an alert to be sent to a user to indicate that the feature of interest is in view of the transducer, and in a fourth block 208 the method comprises sending an instruction to the transducer to trigger the transducer to capture a three-dimensional ultrasound image after a predetermined time interval.

Receiving a data stream of two dimensional images taken using an ultrasound transducer, determining from the data stream that a feature of interest is in view of the transducer, triggering an alert to be sent to a user to indicate that the feature of interest is in view of the transducer, and sending an instruction to the transducer to trigger the transducer to capture a three dimensional ultrasound image after a predetermined time interval were all discussed in detail above with respect to the system 100 and the details therein will be understood to apply equally to embodiments of the method 200.

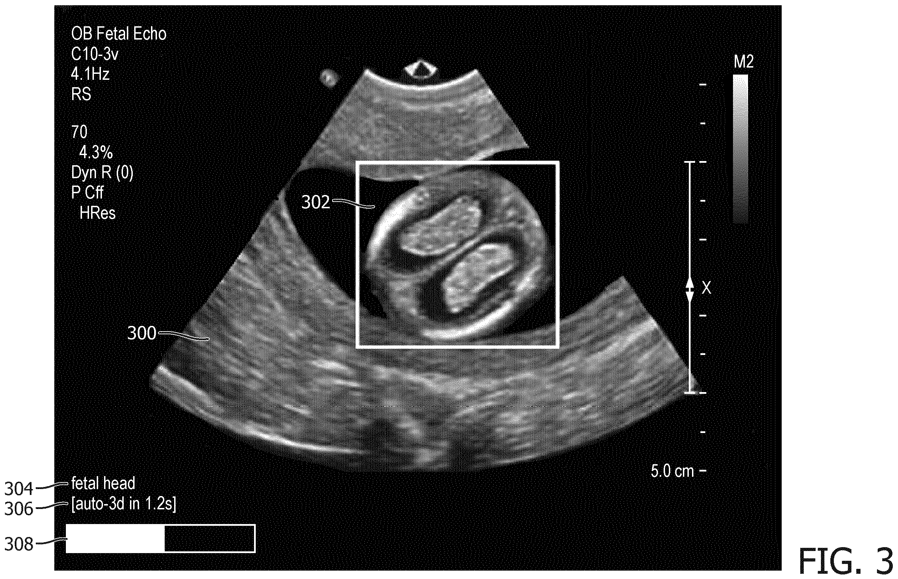

Turning now to Fig. 3, Fig. 3 shows an image as shown on a user display 106 of an ultrasound image according to some embodiments herein. In this embodiment, a data stream of two-dimensional images 300 taken using an ultrasound transducer 108 is received and displayed on a user display 106. When a feature of interest such as the fetal head is determined to be in view of the transducer, an alert is triggered. In this embodiment, the feature of interest is boxed 302, the name of the feature of interest 304 "fetal head" is displayed along with a countdown 306 to commencement of the three-dimensional image capture. A progress bar 308 is also displayed to visually indicate the time remaining until three-dimensional image capture is commenced. The visualization of a bounding box surrounding the feature of interest may provide further guidance to the user.

Once the timer times out (e.g. once the predetermined time interval has passed), three-dimensional imaging of the fetal head is commenced. This creates a user friendly, intuitive workflow for accurate image capture of three-dimensional ultrasound images of anatomical features of interest.

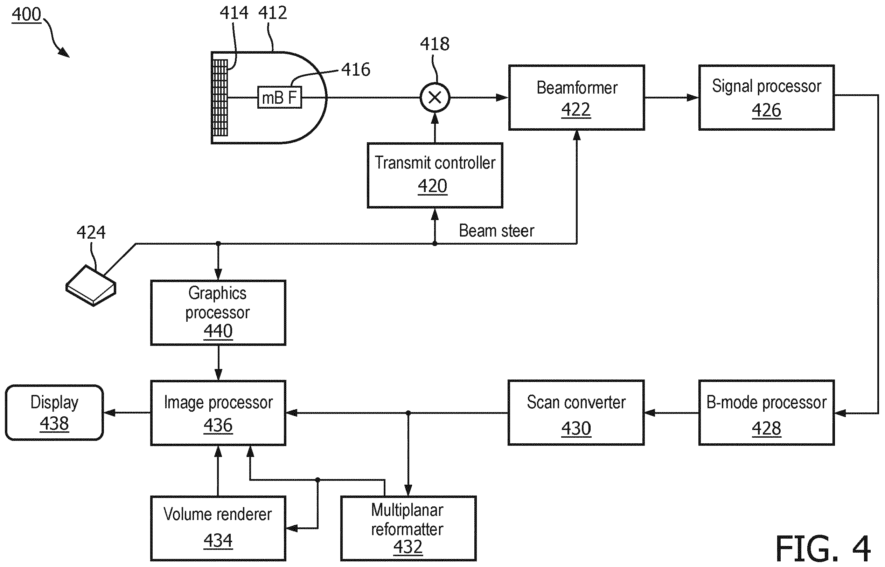

Turning now to Fig. 4, Fig. 4 shows an example embodiment of an ultrasound system 400, constructed according to the principles described herein. One or more components shown in Fig. 4 may be included within a system configured to receive a data stream of two dimensional images taken using an ultrasound transducer, determine from the data stream that a feature of interest is in view of the transducer, trigger an alert to be sent to a user to indicate that the feature of interest is in view of the transducer, and send an instruction to the transducer to trigger the transducer to capture a three dimensional ultrasound image after a predetermined time interval.

For example, any of the above-described functions of the processor 102 may be programmed, e.g., via computer executable instructions, into a processor of the system 400. In some examples, the functions of the processor 102 may be implemented and/or controlled by one or more of the processing components shown in Fig. 4, including for example, the image processor 436.

In the ultrasound imaging system of Fig. 4, ultrasound probe 412 includes a transducer array 414 for transmitting ultrasonic waves into a region of the body and receiving echo information responsive to the transmitted waves. The transducer array 414 may be a matrix array that includes a plurality of transducer elements configured to be individually activated. In other embodiments, the transducer array 414 may comprise a one-dimensional linear array. The transducer array 414 is coupled to a micro-beamformer 416 in the probe 412 which may control the transmission and reception of signals by the transducer elements in the array. In the example shown, the micro-beamformer 416 is coupled by the probe cable to a transmit/receive (T/R) switch 418, which switches between transmission and reception and protects the main beamformer 422 from high energy transmit signals. In some embodiments, the T/R switch 418 and other elements in the system can be included in the transducer probe rather than in a separate ultrasound system base.

In some embodiments herein, ultrasound probe 412 may further comprise a motion detector, to detect motion of the probe, as described above.

The transmission of ultrasonic beams from the transducer array 414 under control of the microbeamformer 416 may be directed by the transmit controller 420 coupled to the T/R switch 418 and the beamformer 422, which receives input, e.g., from the user's operation of the user interface or control panel 424. One of the functions controlled by the transmit controller 420 is the direction in which beams are steered. Beams may be steered straight ahead from (orthogonal to) the transducer array, or at different angles for a wider field of view. The partially beamformed signals produced by the microbeamformer 416 are coupled to a main beamformer 422 where partially beamformed signals from individual patches of transducer elements are combined into a fully beamformed signal.

The beamformed signals are coupled to a signal processor 426. Signal processor 426 may process the received echo signals in various ways, such as bandpass filtering, decimation, I and Q component separation, and harmonic signal separation. Data generated by the different processing techniques employed by the signal processor 426 may be used by a data processor to identify internal structures, e.g., ribs, or anatomical features of a neonate, and parameters thereof.

The signal processor 426 may also perform additional signal enhancement such as speckle reduction, signal compounding, and noise elimination. The processed signals may be coupled to a B-mode processor 428, which can employ amplitude detection for the imaging of structures in the body, including the ribs, the heart, and/or the pleural interface, for example. The signals produced by the B-mode processor are coupled to a scan converter 430 and a multiplanar reformatter 432. The scan converter 430 arranges the echo signals in the spatial relationship from which they were received in a desired image format. For instance, the scan converter 430 may arrange the echo signals into a twodimensional (2D) sector-shaped format. The multiplanar reformatter 432 can convert echoes which are received from points in a common plane in a volumetric region of the body into an ultrasonic image of that plane, as described in U.S. Pat. No. 6,443,896 (Detmer). A volume renderer 434 converts the echo signals of a 3D data set into a projected 3D image as viewed from a given reference point, e.g., as described in U.S. Pat. No. 6,530,885 (Entrekin et al).

The 2D or 3D images are coupled from the scan converter 430, multiplanar reformatter 432, and volume renderer 434 to an image processor 436 for further enhancement, buffering and temporary storage for display on an image display 438.

The graphics processor 440 can generate graphic overlays for display with the ultrasound images. These graphic overlays can contain, for example, a name of a feature of interest detected in the image (e.g. such as the name 304 "fetal head" in Fig. 3); and/or a bounding box surrounding the feature of interest; an alert as described above, indicating for example, that a feature of interest is present and/or the time until commencement of the three dimensional image capture. As noted above, the alert may comprise, for example, a countdown or progress bar (such as the countdown 306 or progress bar 308 shown in Fig. 3)

Graphic overlays may further contain other information, for example, e.g., standard identifying information such as patient name, date and time of the image, imaging parameters, and the like. Graphic overlays may also include one or more signals indicating the target image frame has been obtained and/or the system 400 is in the process of identifying the target image frame. The graphics processor may receive input from the user interface 424, such as a typed patient name. The user interface 424 may also receive input prompting adjustments in the settings and/or parameters used by the system 400. The user interface can also be coupled to the multiplanar reformatter 432 for selection and control of a display of multiple multiplanar reformatted (MPR) images.

The skilled person will appreciate that the embodiment shown in Fig. 4 is an example only and that the ultrasound system 400 may also comprise additional components to those shown in Fig. 4, for example, such as a power supply or battery.

Turning now to other embodiments, in some embodiments there is a computer readable medium, the computer readable medium having computer readable code embodied therein, the computer readable code being configured such that, on execution by a suitable computer or processor, the computer or processor is caused to perform the method 200 described above.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system for recording ultrasound images, the system comprising:
a memory (104) comprising instruction data representing a set of instructions; and
a processor (102) configured to communicate with the memory and to execute the set of instructions, wherein the set of instructions, when executed by the processor, cause the processor to:
receive a data stream of two-dimensional images taken using an ultrasound transducer (108);
determine from the data stream that a feature of interest is in view of the transducer; and
trigger an alert to be sent to a user of the transducer to indicate that the feature of interest is in view of the transducer;
**characterized in that** the processor (102) is further configured to:
receive motion data relating to motion of the transducer (108); and
based on the received motion data, send an instruction to the transducer (108) to trigger the transducer to capture a three-dimensional ultrasound image after a predetermined non-zero time interval.

2. A system as in claim 1, wherein the alert comprises an indication of the time remaining before the three-dimensional ultrasound image capture will be commenced.

3. A system as in claim 2, wherein the indication of the time remaining comprises a countdown or progress bar.

4. A system as in claim 1, 2 or 3, wherein the duration of the predetermined time interval is user configurable.

5. A system as in any one of claims 1 to 4, wherein the processor (102) is configured to send the instruction to the transducer (108) if the motion data indicates that the transducer has been moved by less than a threshold distance during the predetermined time interval.

6. A system as in claim 5, wherein the processor (102) is further configured to refrain from sending the instruction to the transducer (108) if the motion data indicates that the transducer has been moved by more than the threshold distance during the predetermined time interval.

7. A system as in any one of claims 4 to 6, wherein the ultrasound transducer (108) is included in a probe (412) comprising a motion sensor.

8. A system as in any one of claims 1 to 7, wherein the processor (102) is configured to determine from the data stream that a feature of interest is in view of the transducer (108) using a machine learning model that has been trained to identify the feature of interest in an ultrasound image.

9. A system as in claim 8, wherein the processor (102) is further configured to:
receive an indication from the machine learning model that a potential feature is in view of the transducer (108); and
determine whether the potential feature comprises a feature of interest by comparing the potential feature to a list of features of interest.

10. A system as in claim 9, wherein the list of features of interest is derived from a medical protocol describing a plurality of anatomical features to be captured in the three-dimensional ultrasound image according to the protocol.

11. A system as in any one of claims 1 to 10, wherein the data stream comprises medical imaging data and the feature of interest comprises an anatomical feature.

12. A system as in any one of claims 1 to 11, further comprising a user display (106, 438) for displaying the data stream of two-dimensional images and the alert to the user.

13. A computer implemented method (200) of recording ultrasound images, the method comprising:
receiving (202) a data stream of two-dimensional images taken using an ultrasound transducer (108);
determining (204) from the data stream that a feature of interest is in view of the transducer; and
triggering (206) an alert to be sent to a user of the transducer to indicate that the feature of interest is in view of the transducer;
**characterized in that** the method further comprises:
receiving motion data relating to motion of the transducer (108); and
based on the received motion data, sending (208) an instruction to the transducer to trigger the transducer to capture a three-dimensional ultrasound image after a predetermined non-zero time interval.

14. A computer program product comprising a computer readable medium, the computer readable medium having computer readable code embodied therein, the computer readable code being configured such that, on execution by a suitable computer or processor, the computer or processor is caused to perform the method as claimed in claim 13.

## Patentansprüche

1. System zur Aufzeichnung von Ultraschallbildern, wobei das System Folgendes umfasst:
einen Speicher (104), der Anweisungsdaten umfasst, die einen Satz von Anweisungen darstellen; und
einen Prozessor (102), der dazu konfiguriert ist, mit dem Speicher zu kommunizieren und den Satz von Anweisungen auszuführen, wobei der Satz von Anweisungen, wenn er von dem Prozessor ausgeführt wird, den Prozessor zu Folgendem veranlasst:
Empfangen eines Datenstroms von zweidimensionalen Bildern, die mit einem Ultraschallwandler (108) aufgenommen wurden;
Bestimmen aus dem Datenstrom, dass sich ein interessierendes Merkmal im Blickfeld des Wandlers befindet; und
Auslösen einer Benachrichtigung, die an einen Benutzer des Wandlers gesendet werden soll, um anzuzeigen, dass sich das interessierende Merkmal im Blickfeld des Wandlers befindet;
**dadurch gekennzeichnet, dass** der Prozessor (102) weiter konfiguriert ist zum:
Empfangen von Bewegungsdaten bezüglich der Bewegung des Wandlers (108); und
Senden einer Anweisung an den Wandler (108) auf der Grundlage der empfangenen Bewegungsdaten, um den Wandler auszulösen, um ein dreidimensionales Ultraschallbild nach einem vorbestimmten Zeitintervall ungleich Null aufzunehmen.

2. System nach Anspruch 1, wobei die Benachrichtigung eine Angabe über die verbleibende Zeit bis zum Beginn der dreidimensionalen Ultraschallbildaufnahme umfasst.

3. System nach Anspruch 2, wobei die Angabe der verbleibenden Zeit einen Countdown oder einen Fortschrittsbalken umfasst.

4. System nach Anspruch 1, 2 oder 3, wobei die Dauer des vorbestimmten Zeitintervalls vom Benutzer konfigurierbar ist.

5. System nach einem der Ansprüche 1 bis 4, wobei der Prozessor (102) dazu konfiguriert ist, die Anweisung an den Wandler (108) zu senden, wenn die Bewegungsdaten anzeigen, dass der Wandler während des vorbestimmten Zeitintervalls um weniger als eine Schwellendistanz bewegt worden ist.

6. System nach Anspruch 5, wobei der Prozessor (102) weiter dazu konfiguriert ist, das Senden der Anweisung an den Wandler (108) zu unterlassen, wenn die Bewegungsdaten anzeigen, dass der Wandler während des vorbestimmten Zeitintervalls um mehr als die Schwellendistanz bewegt worden ist.

7. System nach einem der Ansprüche 4 bis 6, wobei der Ultraschallwandler (108) in einer Sonde (412) enthalten ist, die einen Bewegungssensor umfasst.

8. System nach einem der Ansprüche 1 bis 7, wobei der Prozessor (102) dazu konfiguriert ist, aus dem Datenstrom zu bestimmen, dass sich ein interessierendes Merkmal im Blickfeld des Wandlers (108) befindet, wobei ein maschinelles Lernmodell verwendet wird, das trainiert wurde, um das interessierende Merkmal in einem Ultraschallbild zu identifizieren.

9. System nach Anspruch 8, wobei der Prozessor (102) weiter konfiguriert ist zum:
Empfangen einer Angabe von dem maschinellen Lernmodell, dass sich ein potenzielles Merkmal im Blickfeld des Messwandlers (108) befindet; und
Bestimmen, ob das potenzielle Merkmal ein interessierendes Merkmal umfasst, indem das potenzielle Merkmal mit einer Liste von interessierenden Merkmalen verglichen wird.

10. System nach Anspruch 9, wobei die Liste der interessierenden Merkmale aus einem medizinischen Protokoll abgeleitet ist, das eine Vielzahl von anatomischen Merkmalen beschreibt, die in dem dreidimensionalen Ultraschallbild gemäß dem Protokoll aufgenommen werden sollen.

11. System nach einem der Ansprüche 1 bis 10, wobei der Datenstrom medizinische Bilddaten umfasst und das interessierende Merkmal ein anatomisches Merkmal umfasst.

12. System nach einem der Ansprüche 1 bis 11, das weiter eine Benutzeranzeige (106, 438) umfasst, um dem Benutzer den Datenstrom zweidimensionalen Bilder und die Benachrichtigung anzuzeigen.

13. Computerimplementiertes Verfahren (200) zur Aufzeichnung von Ultraschallbildern, wobei das Verfahren Folgendes umfasst:
Empfangen (202) eines Datenstroms von zweidimensionalen Bildern, die mit einem Ultraschallwandler (108) aufgenommen wurden;
Bestimmen (204) aus dem Datenstrom, dass sich ein interessierendes Merkmal im Blickfeld des Wandlers befindet; und
Auslösen (206) einer Benachrichtigung, die an einen Benutzer des Wandlers gesendet werden soll, um anzuzeigen, dass sich das interessierende Merkmal im Blickfeld des Wandlers befindet;
**dadurch gekennzeichnet, dass** das Verfahren weiter umfasst:
Empfangen von Bewegungsdaten bezüglich der Bewegung des Wandlers (108); und
Senden (208) einer Anweisung an den Wandler auf der Grundlage der empfangenen Bewegungsdaten, um den Wandler auszulösen, um ein dreidimensionales Ultraschallbild nach einem vorbestimmten Zeitintervall ungleich Null aufzunehmen.

14. Computerprogrammprodukt, das ein computerlesbares Medium umfasst, wobei das computerlesbare Medium einen computerlesbaren Code aufweist, wobei der computerlesbare Code so konfiguriert ist, dass bei Ausführung durch einen geeigneten Computer oder Prozessor der Computer oder Prozessor dazu veranlasst wird, das Verfahren nach Anspruch 13 durchzuführen.

## Revendications

1. Système destiné à enregistrer des images ultrasonores, le système comprenant :
une mémoire (104) comprenant des données d'instructions représentant un ensemble d'instructions ; et
un processeur (102) configuré pour communiquer avec la mémoire et pour exécuter l'ensemble d'instructions, dans lequel l'ensemble d'instructions, lorsqu'il est exécuté par le processeur, amène le processeur à :
recevoir un flux de données d'images bidimensionnelles prises à l'aide d'un transducteur ultrasonore (108) ;
déterminer, à partir du flux de données, qu'une caractéristique d'intérêt est dans le champ de vision du transducteur ; et
déclencher une alerte à envoyer à un utilisateur du transducteur pour indiquer que la caractéristique d'intérêt est dans le champ de vision du transducteur ;
**caractérisé en ce que** le processeur (102) est en outre configuré pour :
recevoir des données de mouvement relatives au mouvement du transducteur (108) ; et
sur la base des données de mouvement reçues, envoyer une instruction au transducteur (108) pour déclencher le transducteur afin de capturer une image ultrasonore tridimensionnelle après un intervalle de temps prédéterminé non nul.

2. Système selon la revendication 1, dans lequel l'alerte comprend une indication du temps restant avant que la capture d'image ultrasonore tridimensionnelle ne commence.

3. Système selon la revendication 2, dans lequel l'indication du temps restant comprend un compte à rebours ou une barre de progression.

4. Système selon la revendication 1, 2 ou 3, dans lequel la durée de l'intervalle de temps prédéterminé est configurable par l'utilisateur.

5. Système selon l'une quelconque des revendications 1 à 4, dans lequel le processeur (102) est configuré pour envoyer l'instruction au transducteur (108) si les données de mouvement indiquent que le transducteur a été déplacé de moins d'une distance seuil pendant l'intervalle de temps prédéterminé.

6. Système selon la revendication 5, dans lequel le processeur (102) est en outre configuré pour s'abstenir d'envoyer l'instruction au transducteur (108) si les données de mouvement indiquent que le transducteur a été déplacé de plus que la distance seuil pendant l'intervalle de temps prédéterminé.

7. Système selon l'une quelconque des revendications 4 à 6, dans lequel le transducteur ultrasonore (108) est inclus dans une sonde (412) comprenant un capteur de mouvement.

8. Système selon l'une quelconque des revendications 1 à 7, dans lequel le processeur (102) est configuré pour déterminer, à partir du flux de données, qu'une caractéristique d'intérêt se trouve dans le champ de vision du transducteur (108) à l'aide d'un modèle d'apprentissage automatique qui a été entraîné pour identifier la caractéristique d'intérêt dans une image ultrasonore.

9. Système selon la revendication 8, dans lequel le processeur (102) est en outre configuré pour :
recevoir une indication du modèle d'apprentissage automatique selon laquelle une caractéristique potentielle se trouve dans le champ de vision du transducteur (108) ; et
déterminer si la caractéristique potentielle comprend ou non une caractéristique d'intérêt en comparant la caractéristique potentielle à une liste de caractéristiques d'intérêt.

10. Système selon la revendication 9, dans lequel la liste de caractéristiques d'intérêt est déduite d'un protocole médical décrivant une pluralité de caractéristiques anatomiques à capturer dans l'image ultrasonore tridimensionnelle selon le protocole.

11. Système selon l'une quelconque des revendications 1 à 10, dans lequel le flux de données comprend des données d'imagerie médicale et la caractéristique d'intérêt comprend une caractéristique anatomique.

12. Système selon l'une quelconque des revendications 1 à 11, comprenant en outre un affichage utilisateur (106, 438) pour afficher le flux de données d'images bidimensionnelles et l'alerte à l'utilisateur.

13. Procédé mis en œuvre par ordinateur (200) destiné à enregistrer des images ultrasonores, le procédé comprenant :
la réception (202) d'un flux de données d'images bidimensionnelles prises à l'aide d'un transducteur ultrasonore (108) ;
la détermination (204), à partir du flux de données, qu'une caractéristique d'intérêt est dans le champ de vision du transducteur ; et
le déclenchement (206) d'une alerte à envoyer à un utilisateur du transducteur pour indiquer que la caractéristique d'intérêt est dans le champ de vision du transducteur ;
**caractérisé en ce que** le procédé comprend en outre :
la réception de données de mouvement relatives au mouvement du transducteur (108) ; et
sur la base des données de mouvement reçues, l'envoi (208) d'une instruction au transducteur pour déclencher le transducteur afin de capturer une image ultrasonore tridimensionnelle après un intervalle de temps prédéterminé non nul.

14. Produit de programme informatique comprenant un support lisible par ordinateur, le support lisible par ordinateur incorporant un code lisible par ordinateur, le code lisible par ordinateur étant configuré de telle sorte que, lors de son exécution par un ordinateur ou un processeur approprié, l'ordinateur ou le processeur soit amené à réaliser le procédé selon la revendication 13.
